# EUROPÄISCHE PATENTANMELDUNG

(11) **EP 4 684 824 A1**
(43) Veröffentlichungstag der Anmeldung: **28.01.2026**
(21) Anmeldenummer: 25191154.1
(22) Anmeldetag: 23.07.2025
(51) Int. Cl.: A61N 1/05, A61N 1/36

(54) **MEDIZINISCHES GERÄT ZUR VERWENDUNG BEI EINER SCHMERZTHERAPIE**

(30) Priorität: 24.07.2024 DE 102024120966
(71) Anmelder: B. Braun Melsungen AG, 34212 Melsungen (DE)
(72) Erfinder: Wildhagen, Jens, 30659 Hannover (DE); Eichenberger, Urs, 6036 Dierikon (CH); Lorenzana, David, 8942 Oberrieden (CH)
(74) Vertreter: Patentanwälte Ruff, Wilhelm, Beier, Dauster & Partner mbB

(57) **Zusammenfassung**

2.1 Ein derartiges medizinisches Gerät aufweisend eine Invasivkomponente mit wenigstens einer Elektrode, die zum Positionieren an einem Nerv und zum Abgeben eines elektrischen Stimulationssignals eingerichtet ist, und einen Signalgenerator, der mit der Elektrode verbunden und zum Erzeugen des Stimulationssignals eingerichtet ist, wobei das Stimulationssignal zum Hemmen einer Erregungsweiterleitung des Nervs eingerichtet ist, ist bekannt.

2.2 Erfindungsgemäß ist die wenigstens eine Elektrode zum Abgeben eines elektrischen Kontrollsignals eingerichtet und der Signalgenerator ist zum Erzeugen des Kontrollsignals eingerichtet, wobei das Kontrollsignal zum Auslösen einer motorischen und/oder sensorischen Reaktion eingerichtet ist, deren Ausprägung von einem Abstand zwischen der Elektrode und dem Nerv abhängig ist.

2.3 Verwendung bei einer Schmerztherapie mittels Neurostimulation

## Beschreibung

Die Erfindung betrifft ein medizinisches Gerät zur Verwendung bei einer Schmerztherapie, aufweisend eine Invasivkomponente mit wenigstens einer Elektrode, die zum Positionieren an einem Nerv und zum Abgeben eines elektrischen Stimulationssignals eingerichtet ist, und einen Signalgenerator, der mit der Elektrode verbunden und zum Erzeugen des Stimulationssignals eingerichtet ist, wobei das Stimulationssignal zum Hemmen einer Erregungsweiterleitung des Nervs eingerichtet ist.

Derartige Geräte sind im Stand der Technik bekannt und werden zur Behandlung akuter oder chronischer Schmerzen verwendet, indem die Weitergabe von Nervenimpulsen durch die Abgabe eines elektrischen Stimulationssignals gehemmt wird, um das Schmerzempfinden des Patienten zu lindern. Hierfür wird die Invasivkomponente des medizinischen Geräts in den Körper eingeführt, wobei die an der Invasivkomponente angeordnete oder ausgebildete Elektrode nervennah positioniert wird. Das elektrische Stimulationssignal wird mittels des Signalgenerators des medizinischen Geräts erzeugt und über die Elektrode abgegeben.

Die Position der Elektrode in Relation zu dem zu stimulierenden Nerv ist maßgeblich für den erzielbaren Therapieeffekt. Bewegt die Elektrode sich während des Stimulationsvorgangs näher an den Nerv, erhöht sich die an den Nerv abgegebene Stimulationsenergie. Entfernt die Elektrode sich, wird hierdurch die auf den Nerv einwirkende Stimulationsenergie reduziert. Solche Abstandsänderungen und damit einhergehende Änderungen der Stimulationsenergie können beispielsweise durch Bewegungen des Patienten verursacht werden.

Aus dem Stand der Technik sind bereits Verfahren zur Regelung der abgegebenen Stimulationsenergie bekannt. Diese Verfahren sehen ein Tracking und eine Korrektur der Stimulationsenergie vor und werden im Bereich der Neurostimulation im Spinalkanal (SCS) verwendet. Hierbei werden beispielsweise die "Ankopplung" des Stimulationssignals an den Nerv und dessen Rückantwort gemessen. Hiervon abhängig wird die Stimulationsenergie des elektrischen Stimulationssignals nachgeregelt.

Die Rückantwort des Nervs kann nur bei einem direkten Kontakt von zwei Elektroden mit dem Nerv gemessen werden. Bei der Stimulation von peripheren Nerven in der sogenannten Single-Shot-Technik kann nicht von einem dauerhaften Kontakt der Elektrode bzw. der Elektroden mit dem Nerv ausgegangen werden.

Aufgabe der Erfindung ist es, ein medizinisches Gerät der eingangs genannten Art bereitzustellen, das eine verbesserte Schmerztherapie ermöglicht.

Diese Aufgabe wird dadurch gelöst, dass die wenigstens eine Elektrode zum Abgeben eines elektrischen Kontrollsignals eingerichtet ist und dass der Signalgenerator zum Erzeugen des Kontrollsignals eingerichtet ist, wobei das Kontrollsignal zum Erzeugen einer motorischen und/oder sensorischen Reaktion eingerichtet ist, deren Ausprägung von einem Abstand zwischen der Elektrode und dem Nerv abhängig ist. Das erfindungsgemäße medizinische Gerät erlaubt folglich die Erzeugung und Abgabe zweier Signale, nämlich zum einen des elektrischen Stimulationssignals und zum anderen des elektrischen Kontrollsignals. Dabei dient das Stimulationssignal der eigentlichen Schmerzunterdrückung, indem es die Erregungsweiterleitung des betreffenden Nervs hemmt. Das Kontrollsignal dient der Positionskontrolle oder auch Abstandskontrolle der Elektrode, indem es zum Auslösen einer motorischen und/oder sensorischen Reaktion des Patienten eingerichtet ist, wobei deren Ausprägung vom Abstand zwischen der Elektrode und dem Nerv abhängt. Je nach Ausprägung der motorischen und/oder sensorischen Reaktion kann das die Schmerztherapie durchführende medizinische Personal (bei einer sensorischen Reaktion durch Rückmeldung des Patienten selbst) erkennen, ob die Elektrode repositioniert werden muss, um eine optimale Schmerztherapie zu ermöglichen. Bei einer schwach ausgeprägten motorischen und/oder sensorischen Reaktion wird regelmäßig ein weiterer distaler Vorschub der Elektrode in Richtung des Nervs notwendig sein. Bei einer stark oder zu stark ausgeprägten motorischen und/oder sensorischen Reaktion wird regelmäßig ein proximales Zurückziehen der Elektrode zur Vergrößerung des Abstands notwendig sein. Die Ausprägung der motorischen und/oder sensorischen Reaktion kann auch von einer Intensität des Kontrollsignals abhängig sein. Bei einer stark oder zu stark ausgeprägten motorischen und/oder sensorischen Reaktion, kann eine Anpassung, im Speziellen Verringerung, der Intensität eines Gesamtsignals bestehend aus Stimulationssignal und Kontrollsignal notwendig sein. Das elektrische Stimulationssignal zur eigentlichen Schmerzunterdrückung weist spezifische Eigenschaften auf, im Speziellen elektrische und/oder signaltechnische Eigenschaften. Diese Eigenschaften sind einer auf dem einschlägigen technischen Gebiet tätigen Fachperson bekannt, so dass eine ausdrückliche Angabe der erwähnten Eigenschaften an dieser Stelle nicht zwingend notwendig ist. Das elektrische Kontrollsignal weist unterschiedliche Eigenschaften auf, im Speziellen unterschiedliche elektrische und/oder unterschiedliche signaltechnische Eigenschaften. Bei einer Ausgestaltung ist die Invasivkomponente ein Katheter, an welchem die wenigstens eine Elektrode angebracht ist. Bei einer weiteren Ausgestaltung ist die Invasivkomponente eine Stimulationskanüle, an welcher die wenigsten eine Elektrode angebracht ist. Bei einer weiteren Ausgestaltung ist die Invasivkomponente ein Draht und bildet gleichsam die wenigstens eine Elektrode aus oder umgekehrt. Bei einer Ausgestaltung weist das medizinische Gerät eine einzige Elektrode auf, die sowohl zur Abgabe des Stimulationssignals als auch zur Abgabe des Kontrollsignals eingerichtet ist. Bei einer weiteren Ausgestaltung sind wenigstens zwei Elektroden vorhanden, wobei eine erste der beiden Elektroden zur Abgabe des Stimulationssignals und eine zweite der beiden Elektroden zur Abgabe des Kontrollsignals eingerichtet ist. Alternativ oder zusätzliche kann eine erste Elektrode der wenigstens zwei Elektroden als Kathode und eine zweite Elektrode der wenigstens zwei Elektroden als Anode verwendet werden. Die Invasivkomponente mitsamt der Elektrode ist zum Einführen in den Körper des Patienten eingerichtet. Demgegenüber ist der Signalgenerator eine extrakorporale Komponente des medizinischen Geräts, die drahtgebunden mit der wenigstens einen Elektrode der Invasivkomponente verbunden ist. Mit anderen Worten: Der Signalgenerator ist zum Verbleib außerhalb des Patientenkörpers vorgesehen. Bei einer Ausgestaltung weist das medizinische Gerät eine Erfassungseinrichtung auf, die zum Erfassen der mittels des Kontrollsignals ausgelösten motorischen und/oder sensorischen Reaktion und im Speziellen deren Ausprägung eingerichtet ist. Eine solche Erfassungseinrichtung ist bei einer weiteren Ausgestaltung zudem dazu eingerichtet, in Abhängigkeit der erfassten Ausprägung der motorischen und/oder sensorischen Reaktion ein Signal zu erzeugen, das die Ausprägung der motorischen und/oder sensorischen Reaktion repräsentiert. Vorzugsweise ist das Signal ein akustisches, optisches und/oder auf sonstige Weise von medizinischem Personal wahrnehmbares Signal, auf dessen Grundlage das medizinische Personal den Abstand der Elektrode zu dem Nerv verringern oder vergrößern kann. Bei einer weiteren Ausgestaltung ist keine solche Erfassungseinrichtung vorgesehen. Stattdessen wird die Ausprägung der motorischen und/oder sensorischen Reaktion durch das medizinische Personal selbst erfasst, beispielsweise visuell, taktil und/oder auf sonstige Weise.

In Ausgestaltung der Erfindung ist das elektrische Stimulationssignal derart eingerichtet, dass es keine, insbesondere keine praktisch erhebliche, motorische und/oder sensorische Reaktion auslöst, und das Kontrollsignal ist derart eingerichtet, dass es keine Hemmung der Erregungsweiterleitung erzeugt. Mit anderen Worten: Bei dieser Ausgestaltung gibt es keine oder jedenfalls keine praktisch erhebliche Schnittmenge hinsichtlich der durch das betreffende Signal erzeugten physiologischen Reaktion(en). Das Stimulationssignal hat bei dieser Ausgestaltung allenfalls einen geringen, vorzugsweise keinen, Einfluss auf die motorische und/oder sensorische Reaktion. Hierdurch wird vermieden, dass das Kontrollsignal in unerwünschter Weise die Wirkung des Stimulationssignals überlagert und hierdurch beeinträchtigt. Umgekehrt wird auch vermieden, dass das Stimulationssignal die Wirkung des Kontrollsignals beeinträchtigt und verfälscht.

In weiterer Ausgestaltung der Erfindung weist das Stimulationssignal eine Frequenz auf, die um wenigstens einen Faktor 0,5x10³, bevorzugt wenigstens 1x10³, weiter bevorzugt 1,5x10³, größer ist als eine Frequenz des Kontrollsignals. Bei dieser Ausgestaltung ist das Stimulationssignal in Relation zu dem Kontrollsignal hochfrequent. Umgekehrt ist das Kontrollsignal in Relation zu dem Stimulationssignal niederfrequent. Durch eine solche Frequenzwahl kann vermieden werden, dass es zu unerwünschten Überschneidungen in der physiologischen Wirkung kommt (einerseits Hemmung der Erregungsweiterleitung, andererseits Auslösen der motorischen und/oder sensorischen Reaktion).

In weiterer Ausgestaltung sind eine/die Frequenz des Stimulationssignals und eine/die Frequenz des Kontrollsignals ähnlich. Bevorzugt unterscheiden sich die Frequenzen um einen Faktor von maximal 5, weiter bevorzugt von maximal 2, weiter bevorzugt von maximal 1,1. Dabei wird das Stimulationssignal derart erzeugt, dass es keine oder jedenfalls keine praktisch erhebliche motorische und/oder sensorische Reaktion auslöst, d.h. unterhalb einer Wahrnehmungsschwelle liegt.

In weiterer Ausgestaltung der Erfindung weist das Kontrollsignal eine Frequenz von 0,1 Hz bis 10 Hz, bevorzugt von 0,5 Hz bis 5 Hz, weiter bevorzugt von 1 Hz bis 2 Hz, auf und/oder das Stimulationssignal weist eine Frequenz von wenigstens 5 kHz, bevorzugt von wenigstens 10 kHz, weiter bevorzugt von wenigstens 20 kHz, auf. Die vorgenannten Wertebereiche für die Frequenzen des Kontrollsignals und des Stimulationssignals haben sich als besonders vorteilhaft erwiesen. Die vorstehenden Wertebereiche für die Frequenz des Kontrollsignals sind insbesondere dann vorteilhaft, wenn eine motorische Reaktion ausgelöst werden soll (motorisches Kontrollsignal). Sofern eine sensorische Reaktion/Wahrnehmung ausgelöst werden soll (sensorisches Kontrollsignal) weist das Kontrollsignal vorzugsweise eine Frequenz von 3 Hz bis 5 Hz auf.

In weiterer Ausgestaltung der Erfindung weist das Stimulationssignal eine Frequenz von 0,1 Hz bis 200 Hz, bevorzugt von 0,5 Hz bis 50 Hz, weiter bevorzugt von 3 Hz bis 5 Hz auf, insbesondere wobei das Stimulationssignal eine Signalstärke aufweist, die unterhalb der Wahrnehmungsschwelle liegt.

In weiterer Ausgestaltung der Erfindung weist das Kontrollsignal eine Amplitude von 0,01 mA bis 20 mA, bevorzugt von 0,1 mA bis 10 mA, weiter bevorzugt von 0,5 mA bis 3 mA, auf. Die vorgenannten Wertebereiche für die Amplitude der Stromstärke des Kontrollsignals haben sich als besonders vorteilhaft erwiesen. Die vorstehenden Wertebereiche für die Amplitude des Kontrollsignals sind insbesondere dann vorteilhaft, wenn eine motorische Reaktion ausgelöst werden soll (motorisches Kontrollsignal). Sofern eine sensorische Reaktion/Wahrnehmung ausgelöst werden soll (sensorisches Kontrollsignal) weist das Kontrollsignal vorzugsweise eine Amplitude von 0,03 mA bis 60 mA, bevorzugt von 0,3 mA bis 30 mA, weiter bevorzugt von 1,5 mA bis 9 mA, auf.

In weiterer Ausgestaltung der Erfindung ist der Signalgenerator dazu eingerichtet, das Kontrollsignal als gepulstes Signal zu erzeugen und das Stimulationssignal als gepulstes Signal zu erzeugen. Das Stimulationssignal wird dabei mit einer Amplitude unterhalb der Wahrnehmungsschwelle erzeugt, das heißt so, dass es keine oder jedenfalls keine praktisch erhebliche motorische und/oder sensorische Reaktion auslöst.

In weiterer Ausgestaltung der Erfindung ist der Signalgenerator dazu eingerichtet, das Kontrollsignal als gepulstes Signal zu erzeugen und das Stimulationssignal als kontinuierliches Signal zu erzeugen. Vorzugsweise wird das Stimulationssignal als breitbandiges und/oder hochfrequentes Signal erzeugt. Bei dieser Ausgestaltung der Erfindung ist folglich eine zeitkontinuierliche Abgabe des Stimulationssignals vorgesehen, wohingegen das Kontrollsignal gepulst und folglich zeitdiskontinuierlich oder auch zu diskreten Zeitpunkten abgegeben wird. Eine solche einerseits kontinuierliche Abgabe des Stimulationssignals und andererseits gepulste Abgabe des Kontrollsignals hat sich als besonders vorteilhaft erwiesen.

In weiterer Ausgestaltung der Erfindung weist das Kontrollsignal eine Impulsbreite von 0,01 ms bis 20 ms, bevorzugt von 0,05 ms bis 10 ms, weiter bevorzugt von 0,1 ms bis 1 ms, auf. Die vorgenannten Wertebereiche für die Impulsbreite des Kontrollsignals gehen mit besonderen Vorteilen einher. Die vorstehenden Wertebereiche für die Impulsbreite des Kontrollsignals sind insbesondere dann vorteilhaft, wenn eine motorische Reaktion ausgelöst werden soll (motorisches Kontrollsignal). Sofern eine sensorische Reaktion/Wahrnehmung ausgelöst werden soll (sensorisches Kontrollsignal) weist das Kontrollsignal vorzugsweise eine Impulsbreite von 0,2 ms auf.

In weiterer Ausgestaltung der Erfindung ist der Signalgenerator dazu eingerichtet, das Kontrollsignal in wenigstens einem Burst mit einer Impulsanzahl von 1 bis 100 Impulsen, bevorzugt von 1 bis 10 Impulsen, weiter bevorzugt von 1 bis 5 Impulsen, zu erzeugen. Bei dieser Ausgestaltung sind folglich eine stoßweise Erzeugung und Abgabe des Kontrollsignals vorgesehen. Die Erzeugung und Abgabe erfolgen in Form wenigstens eines Bursts, d. h. einer Abfolge von Einzelimpulsen, wobei die Abfolge eine definierte Anzahl an Einzelimpulsen aufweist. Vorzugsweise weisen die Impulse jeweils eine identische Impulsbreite, d.h. Impulsdauer, auf. Weiter vorzugsweise ist zwischen den Impulsen jeweils eine definierte (kurze) Pausendauer vorgesehen. Sofern mehrere aufeinanderfolgende Bursts erzeugt und abgegeben werden, ist eine zwischen den Bursts vorgesehene (längere) Pausendauer vorgesehen. Bei dieser Ausgestaltung der Erfindung erfolgen die Erzeugung und Abgabe des Kontrollsignals in Relation zu dem Stimulationssignal zeitlich stark begrenzt. Hierdurch kann in nochmals verbesserter Weise vermieden werden, dass es zu einer unerwünschten Überlagerung der mittels der beiden Signale erzeugten physiologischen Reaktionen kommt. Außerdem kann nicht unter allen Umständen ausgeschlossen werden, dass das Kontrollsignal und die mit diesem ausgelöste motorische und/oder sensorische Reaktion ein gewisses Unbehagen bei dem Patienten hervorruft. Durch die zeitlich eingegrenzte/eingeschränkte Signalabgabe in Form des wenigstens einen Pulses und/oder einen Bursts können solche potenziell unangenehmen Begleiterscheinungen des Kontrollsignals für den Patienten auf ein Minimum reduziert werden.

In weiterer Ausgestaltung der Erfindung ist der Signalgenerator dazu eingerichtet, das Kontrollsignal in wenigstens zwei aufeinanderfolgenden Bursts zu erzeugen, wobei eine Pausendauer zwischen den Bursts wenigstens 10 s, bevorzugt wenigstens 15 s, weiter bevorzugt wenigstens 20 s, beträgt. Durch eine solche Wahl der Pausendauer wird eine übermäßige Beanspruchung des Patienten durch das Kontrollsignal und durch die mittels des Kontrollsignals ausgelösten motorischen und/oder sensorischen Reaktionen vermieden. Zudem geht diese Ausgestaltung der Erfindung von der Überlegung aus, dass eine Kontrolle des Abstands der Elektrode nicht unbedingt zeitkontinuierlich erfolgen muss. Regelmäßig wird eine Kontrolle in gewissen Zeitabständen bereits ausreichend sein. Diese Ausgestaltung erlaubt eine Kontrolle in solchen Zeitabständen, wobei die Zeitabstände für die Kontrolle des Abstands durch die Pausendauer zwischen den Bursts definiert sind.

In weiterer Ausgestaltung der Erfindung ist der Signalgenerator dazu eingerichtet, das Kontrollsignal und das Stimulationssignal alternierend zu erzeugen. Dabei ist die Elektrode zur alternierenden oder gleichzeitigen Abgabe der beiden Signale eingerichtet.

In weiterer Ausgestaltung der Erfindung ist der Signalgenerator dazu eingerichtet, das Kontrollsignal und das Stimulationssignal gleichzeitig zu erzeugen. Bei dieser Ausgestaltung ist die Elektrode dementsprechend auch zur gleichzeitigen Abgabe der beiden Signale eingerichtet.

Die Erfindung betrifft zudem ein Verfahren zur Abstandskontrolle einer Elektrode bei einer Schmerztherapie mittels elektrischer Neurostimulation. Das erfindungsgemäße Verfahren weist folgende Schritte auf: Erzeugen eines elektrischen Kontrollsignals, wobei das Kontrollsignal mittels eines Signalgenerators eines medizinischen Geräts erzeugt wird; Abgeben des erzeugten Kontrollsignals, wobei das Kontrollsignal über eine mit dem Signalgenerator verbundene Elektrode einer Invasivkomponente des medizinischen Geräts abgegeben wird, wobei die Elektrode in einem Abstand zu einem Nerv angeordnet ist, und wobei das Kontrollsignal unter Einwirkung auf den Nerv eine motorische und/oder sensorische Reaktion auslöst, deren Ausprägung von dem Abstand der Elektrode abhängig ist; Erfassen der Ausprägung der motorischen und/oder sensorischen Reaktion, wobei die Ausprägung der motorischen und/oder sensorischen Reaktion mittels einer Erfassungseinrichtung des medizinischen Geräts und/oder durch medizinisches Personal erfasst wird; Kontrollieren des Abstands der Elektrode in Abhängigkeit der erfassten Ausprägung der motorischen und/oder sensorischen Reaktion. Die mit dem erfindungsgemäßen Verfahren einhergehenden Vorteile korrespondieren mit den Vorteilen des erfindungsgemäßen medizinischen Geräts. Zur Vermeidung von Wiederholungen wird auf die diesbezügliche Offenbarung des medizinischen Geräts verwiesen, die mutatis mutandis auch für das erfindungsgemäße Verfahren gilt. Weitere Ausgestaltungen des erfindungsgemäßen Verfahrens ergeben sich aus den Merkmalen des erfindungsgemäßen medizinischen Geräts und dessen Ausgestaltungen.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Blockdarstellung eine Ausführungsform eines erfindungsgemäßen medizinischen Geräts, das zur Verwendung bei einer Schmerztherapie vorgesehen ist,
- Fig. 2: ein schematisch vereinfachtes Diagramm zur Verdeutlichung von Eigenschaften von mittels des medizinischen Geräts erzeugten und abgegebenen elektrischen Signalen und
- Fig. 3: in schematischer Blockdarstellung eine Ausführungsform eines erfindungsgemäßen Verfahrens zur Abstandskontrolle einer Elektrode bei einer Schmerztherapie.

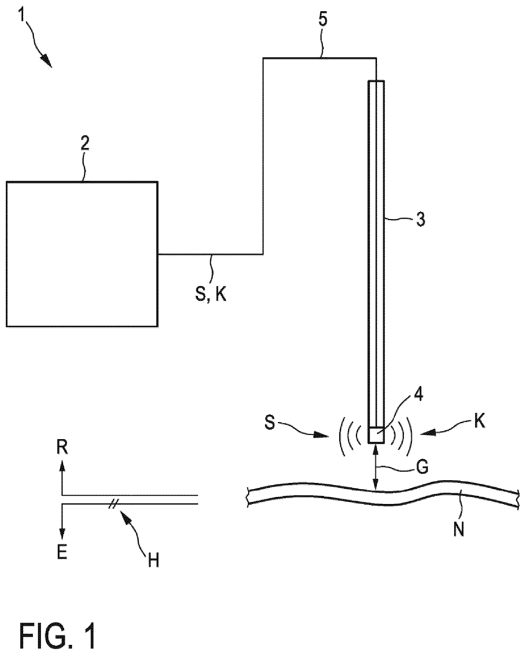

Gemäß Fig. 1 ist ein medizinisches Gerät 1 zur Verwendung bei einer Schmerztherapie vorgesehen und weist einen Signalgenerator 2, eine Invasivkomponente 3 und eine Elektrode 4 auf.

Der Signalgenerator 2 ist zum Erzeugen eines elektrischen Stimulationssignals S und eines elektrischen Kontrollsignals K eingerichtet. Die Elektrode 4 ist zum Abgeben des Stimulationssignals S und des Kontrollsignals K eingerichtet. Der Signalgenerator 2 und die Elektrode 4 sind mittels einer Signalleitung 5 miteinander verbunden. Die Signalleitung 5 dient einer Übertragung der erzeugten Signale S, K von dem Signalgenerator 2 zu der Elektrode 4. Die Signalleitung 5 kann eine drahtgebundene oder eine drahtlose Übertragungsleitung sein, wobei die drahtgebundene Übertragung bevorzugt ist.

Die Elektrode 4 ist an einem nicht näher bezeichneten distalen Ende der Invasivkomponente 3 angeordnet und/oder ausgebildet. Die Invasivkomponente 3 kann gleichsam als Elektrode 4 aufgefasst werden und umgekehrt. Mit anderen Worten: Die Invasivkomponente 3 ist optional und nicht bei sämtlichen Ausführungsformen vorhanden. Im einfachsten Fall weist das medizinische Gerät lediglich den Signalgenerator und die Elektrode auf, die beispielsweise in Form eines Signaldrahts ausgestaltet sein kann, der ausgehend von dem Signalgenerator längserstreckt ist.

Die Invasivkomponente 3 mitsamt der Elektrode 4 ist zum Einführen in den Körper eines Patienten eingerichtet. Die Invasivkomponente kann beispielsweise ein Katheter, eine Stimulationskanüle oder dergleichen sein. Demgegenüber ist der Signalgenerator 2 eine extrakorporale Komponente des medizinischen Geräts 1 und folglich zum Verbleib außerhalb des Patientenkörpers eingerichtet.

In der Verwendung des medizinischen Geräts 1 ist die Elektrode 4 im Bereich eines Nervs N positioniert. In der in Fig. 1 gezeigten exemplarischen Verwendungssituation ist die Elektrode 4 in einem Abstand G von dem Nerv N positioniert.

Das mittels des Signalgenerators 2 erzeugte und mittels der Elektrode 4 abgegebene Stimulationssignal S ist zum Hemmen einer Erregungsweiterleitung E des Nervs N eingerichtet. Die besagte Hemmung H ist in Fig. 1 schematisch stark vereinfacht als eine Art Unterbrechung der Erregungsweiterleitung E dargestellt. Die Hemmung H der Erregungsweiterleitung E bewirkt eine Schmerzlinderung oder auch -unterdrückung.

Das Kontrollsignal K ist dazu eingerichtet, unter Einwirkung auf den Nerv N eine motorische Reaktion R auszulösen. Eine Ausprägung oder auch Stärke der motorischen Reaktion hängt von dem Abstand G zwischen der Elektrode 4 und dem Nerv N ab. Entsprechendes gilt für die Wirksamkeit der Schmerzlinderung mittels des Stimulationssignals S. Eine zu starke Verringerung des Abstands G kann dazu führen, dass eine mittels des Stimulationssignals S auf den Nerv N übertragene elektrische Energie zu groß wird. Umgekehrt kann eine zu starke Vergrößerung des Abstands G dazu führen, dass die elektrische Stimulationsenergie des Stimulationssignals S zu gering wird. Beides kann zu einer Beeinträchtigung der Wirksamkeit der Schmerztherapie führen.

Das medizinische Gerät 1 erlaubt eine Kontrolle des Abstands G, insbesondere indem die motorische Reaktion R erfasst und der Abstand G dementsprechend nachjustiert wird. Das Erfassen der motorischen Reaktion R kann im einfachsten Fall durch medizinisches Personal, d. h. einen Benutzer des medizinischen Geräts 1, erfolgen. Anhand der beobachteten motorischen Reaktion R, genauer: deren Ausprägung/Stärke, kann der Benutzer beurteilen, ob die Elektrode 4 zum Erreichen eines optimalen Therapieeffekts relativ zu dem Nerv N verlagert werden muss, um den Abstand G und die von dem Abstand G abhängige Hemmung H zu optimieren. Alternativ oder zusätzlich kann der Therapieeffekt durch eine Anpassung, insbesondere Verstärkung, des Stimulationssignals S angepasst werden.

Bei einer in den Figuren nicht gezeigten Ausführungsform weist das medizinische Gerät eine Erfassungseinrichtung auf, die zum sensorbasierten Erfassen der motorischen Reaktion, im Speziellen deren Ausprägung, und zum Erzeugen eines Signals eingerichtet ist, welches die Ausprägung der erfassten motorischen Reaktion repräsentiert. In Abhängigkeit des mittels der Erfassungseinrichtung erzeugten Signals kann der Abstand entsprechend nachgeregelt werden.

Es gibt aber auch Anwendungsfälle, bei denen rein sensorische Nerven stimuliert werden. Bei der Neuromodulation von rein sensorischen Nerven kann keine Kontrolle durch eine motorische Reaktion erfolgen. In diesem Fall kann stattdessen von einer sensorischen Reaktion und dementsprechend von einem "sensorischen Kontrollsignal" gesprochen werden. Die Positionskontrolle erfolgt in diesem Fall nicht anhand der motorischen Reaktionen, sondern anhand des Patientenfeedbacks im Hinblick auf eine durch das Kontrollsignal verursachte sensorische Wahrnehmung. Der Patient spürt das (sensorische) Kontrollsignal und gibt dem behandelnden Anästhesisten eine entsprechende Rückmeldung: Spürt der Patient nichts, ist der Abstand/die Intensität falsch gewählt. Spürt der Patient das Kontrollsignal zu stark, wird die Intensität verringert/der Abstand erhöht. Die Begriffe "sensorisches Kontrollsignal" und "motorisches Kontrollsignal" werden im Rahmen dieser Offenbarung unter dem Begriff "Kontrollsignal" zusammengefasst, sofern nichts anderes beschrieben ist.

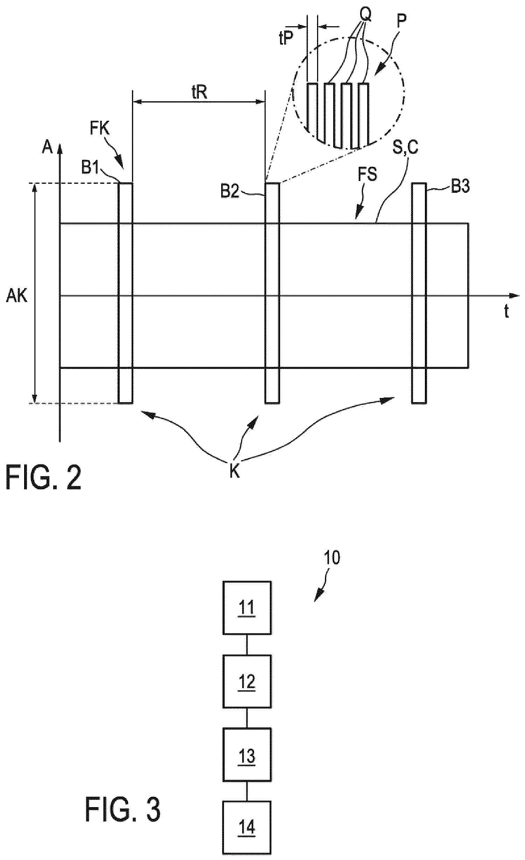

Das Stimulationssignal S und das Kontrollsignal K weisen unterschiedliche Eigenschaften auf, insbesondere unterschiedliche elektrische und/oder unterschiedliche signaltechnische Eigenschaften. Diese unterschiedlichen Eigenschaften werden nachfolgend unter Bezugnahme auf Fig. 2 erläutert.

In Fig. 2 ist ein schematisch stark vereinfachter Zeitverlauf der beiden Signale S, K gezeigt, wobei in dem dargestellten Diagramm eine Amplitude A über der Zeit t dargestellt ist. Dabei bezieht sich die Amplitude A vorliegend auf eine Stromstärke der jeweiligen Signale S, K.

Bei der gezeigten Ausführungsform ist das Kontrollsignal K ein niederfrequentes Signal mit einer Frequenz FK. Das Stimulationssignal S ist ein hochfrequentes Signal mit einer Frequenz FS. Die Frequenz FS ist bei der gezeigten Ausführungsform um etwa 2,5x10³ größer als die Frequenz FK des Kontrollsignals K. Im Speziellen beträgt die Frequenz FK des Kontrollsignals K vorliegend 1 Hz. Die Frequenz FS des Stimulationssignals beträgt vorliegend 25 kHz.

Bei einer in den Figuren nicht gezeigten Ausführungsform weist das Stimulationssignal eine Frequenz von 0,1 Hz bis 200 Hz, bevorzugt von 0,5 Hz bis 50 Hz, weiter bevorzugt von 3 Hz bis 5 Hz auf, insbesondere wobei das Stimulationssignal eine Signalstärke aufweist, die unterhalb der Wahrnehmungsschwelle liegt.

Weiter beträgt eine Amplitude AK des Kontrollsignals K vorliegend 1,5 mA. Bei in den Figuren nicht gezeigten Ausführungsformen beträgt die Amplitude des Kontrollsignals zwischen 0,01 mA und 20 mA.

Wie weiter in Fig. 2 gezeigt ist, handelt es sich bei dem Stimulationssignal S um ein kontinuierliches Signal C. Demgegenüber ist das Kontrollsignal K ein gepulstes Signal P. Mit anderen Worten: Das Stimulationssignal S wird über der Zeit t kontinuierlich oder jedenfalls quasikontinuierlich abgegeben. Demgegenüber wird das Kontrollsignal K gepulst oder auch getaktet und/oder mit zeitlichen Unterbrechungen versehen erzeugt und abgegeben.

Wie in dem vergrößerten Bereich in Fig. 2 gezeigt ist, beträgt eine Impulsbreite tP des Kontrollsignals K vorliegend 0,5 ms. Bei in den Figuren nicht gezeigten Ausführungsformen beträgt die Impulsbreite des Kontrollsignals von 0,01 ms bis 20 ms.

Wie weiter in Fig. 2 gezeigt ist, erfolgt die Erzeugung und Abgabe des Kontrollsignals K in mehreren Bursts oder mit anderen Worten stoßweise, wobei vorliegend exemplarisch mehrere Bursts B1, B2, B3 gezeigt sind. Diese können auch als erster Burst B1, zweiter Burst B2 und dritter Burst B3 bezeichnet werden. Es versteht sich, dass die gezeigte Anzahl der Bursts rein exemplarisch ist. Je nach Dauer der Schmerztherapie können selbstverständlich auch mehr oder sogar deutlich mehr als die vorliegend exemplarisch gezeigten drei Burst erzeugt und abgegeben werden.

Jeder der gezeigten Bursts B1, B2, B3 weist eine definierte Anzahl an Impulsen Q auf, wobei vorliegend je Burst vier Impulse abgegeben werden. Auch diese Impulsanzahl ist exemplarisch. Bei in den Figuren nicht gezeigten Ausführungsformen beträgt die Impulsanzahl je Burst ein bis einhundert Impulse, bevorzugt ein bis zehn Impulse, weiter bevorzugt ein bis fünf Impulse.

Zwischen den Bursts B1, B2, B3 ist jeweils eine Pausendauer tR vorgesehen. Diese beträgt vorliegend 20 s. Bei in den Figuren nicht gezeigten Ausführungsformen beträgt die Pausendauer wenigstens 10 s, bevorzugt wenigstens 15 s und weiter bevorzugt wenigstens 20 s.

In Fig. 3 ist schematisch vereinfacht ein Verfahren 10 zur Abstandskontrolle der Elektrode 4 bei der Verwendung des medizinischen Geräts 1 gezeigt. Das Verfahren sieht ein Erzeugen 11 des elektrischen Kontrollsignals K vor, wobei das Kontrollsignal K mittels des Signalgenerators 2 erzeugt wird. Das Verfahren sieht weiter ein Abgeben 12 des erzeugten Kontrollsignals K vor, wobei das Kontrollsignal K über die mittels der Signalleitung 5 mit dem Signalgenerator 2 verbundene Elektrode 4 der Invasivkomponente 3 abgegeben wird. Die Elektrode 4 ist dabei in dem bereits erwähnten Abstand G zu dem Nerv N angeordnet. Das Kontrollsignal K löst unter Einwirkung auf den Nerv N die bereits erwähnte motorische und/oder sensorische Reaktion R aus, deren Ausprägung oder auch Stärke von dem Abstand G abhängt. Das Verfahren sieht weiter ein Erfassen 13 der Ausprägung der motorischen und/oder sensorischen Reaktion R vor. Das Erfassen 13 erfolgt entweder mittels der zuvor erwähnten optionalen Erfassungseinrichtung oder durch das medizinische Personal selbst. Weiter sieht das Verfahren 10 ein Kontrollieren 14 des Abstands G in Abhängigkeit der erfassten Ausprägung der motorischen und/oder sensorischen Reaktion R vor. Das Kontrollieren 14 erfolgt im einfachsten Fall durch das medizinische Personal selbst. Alternativ kann das medizinische Gerät eine optionale Kontrolleinrichtung aufweisen, die den Abstand automatisch kontrolliert und optional nachregelt. Diese Kontrolle und optionale Nachregelung können beispielsweise in Abhängigkeit eines mittels der optionalen Erfassungseinrichtung erzeugten Signals erfolgen, das die Ausprägung der motorischen und/oder sensorischen Reaktion repräsentiert.

Bei weiteren Ausführungsformen haben sich für die Impulsbreite, die Frequenz und die Stromstärke des Kontrollsignals und des Stimulationssignals folgende Wertebereiche als vorteilhaft erwiesen:

| | | Kontrollsignal | | Stimulationssignal | |
|---|---|---|---|---|---|
| | | sensorische Reaktion | motorisch Reaktion | hochfrequent | niederfrequent |
| Impulsbreite | | 0,01ms - 20ms | 0,01ms - 20ms | - | 0,01ms - 250ms |
| | bevorzugt | 0,05ms - 10ms | 0,05ms - 10ms | | 0,1ms 100ms |
| | weiter bevorzugt | 0,1ms - 0,5ms, ideal 0,2ms | 0,1ms - 0,5ms | | 0,1ms - 0,5ms, ideal 0,2ms |
| Frequenz | | 0,1Hz-100Hz | 0,1Hz-10Hz | >5kHz | 0,1Hz-200Hz |
| | bevorzugt | 0,5Hz-50Hz | 0,5Hz-5Hz | >10kHz | 0,5Hz-50Hz |
| | weiter bevorzugt | 3-5 Hz | 1-2 Hz | >20kHz | 3-5Hz |
| Stromstärke | | 0,03mA - 60mA | 0,01mA - 20mA | - | geringerer als das Kontrollsignal |
| | bevorzugt | 0,3mA - 30mA | 0,1mA - 19mA | | |
| | weiter bevorzugt | 1,5mA - 9mA | 0,5mA - 3mA | | |

## Patentansprüche

1. Medizinisches Gerät (1) zur Verwendung bei einer Schmerztherapie, aufweisend
eine Invasivkomponente (3) mit wenigstens einer Elektrode (4), die zum Positionieren an einem Nerv (N) und zum Abgeben eines elektrischen Stimulationssignals (S) eingerichtet ist, und
einen Signalgenerator (2), der mit der Elektrode (4) verbunden und zum Erzeugen des Stimulationssignals (S) eingerichtet ist,
wobei das Stimulationssignal (S) zum Hemmen (H) einer Erregungsweiterleitung (E) des Nervs (N) eingerichtet ist,
**dadurch gekennzeichnet, dass** die wenigstens eine Elektrode (4) zum Abgeben eines elektrischen Kontrollsignals (K) eingerichtet ist und der Signalgenerator (2) zum Erzeugen des Kontrollsignals (K) eingerichtet ist, wobei das Kontrollsignal (K) zum Auslösen einer motorischen und/oder sensorischen Reaktion (R) eingerichtet ist, deren Ausprägung von einem Abstand (G) zwischen der Elektrode (4) und dem Nerv (N) abhängig ist.

2. Medizinisches Gerät (1) nach Anspruch 1, wobei das elektrische Stimulationssignal (S) nicht dazu eingerichtet ist, eine motorische und/oder sensorische Reaktion auszulösen, und wobei das elektrische Kontrollsignal (K) nicht dazu eingerichtet ist, die Erregungsweiterleitung (E) des Nervs (N) zu hemmen.

3. Medizinisches Gerät (1) nach Anspruch 1 oder 2, wobei eine Frequenz (FS) des Stimulationssignals (S) um einen Faktor von wenigstens 0,5x10³, bevorzugt von 1,0x10³, weiter bevorzugt von 1,5x10³, größer ist als eine Frequenz (FK) des Kontrollsignals (K).

4. Medizinisches Gerät (1) nach Anspruch 1 oder 2, wobei eine Frequenz (FS) des Stimulationssignal (S) und eine Frequenz (FK) des Kontrollsignals (K) ähnlich sind.

5. Medizinisches Gerät (1) nach einem der vorhergehenden Ansprüche, wobei das Kontrollsignal (K) eine Frequenz (FK) von 0,1 Hz bis 10 Hz, bevorzugt von 0,5 Hz bis 5 Hz, weiter bevorzugt von 1 Hz bis 2 Hz, aufweist, und/oder wobei das Stimulationssignal (S) eine Frequenz (FS) von wenigstens 5 kHz, bevorzugt von wenigstens 10 kHz, weiter bevorzugt von wenigstens 20 kHz, aufweist.

6. Medizinisches Gerät (1) nach einem der Ansprüche 1 bis 4, wobei das Stimulationssignal (S) eine Frequenz (FK) von 0,1 Hz bis 200 Hz, bevorzugt von 0,5 Hz bis 50 Hz, weiter bevorzugt von 3 Hz bis 5 Hz aufweist, insbesondere wobei das Stimulationssignal (S) eine Signalstärke aufweist, die unterhalb der Wahrnehmungsschwelle liegt.

7. Medizinisches Gerät (1) nach einem der vorhergehenden Ansprüche, wobei das Kontrollsignal (K) eine Amplitude (AK) von 0,01 mA bis 20 mA, bevorzugt von 0,1 mA bis 10 mA, weiter bevorzugt von 0,5 mA bis 3 mA, aufweist.

8. Medizinisches Gerät (1) nach einem der vorhergehenden Ansprüche, wobei der Signalgenerator (2) dazu eingerichtet ist, das Kontrollsignal (K) als gepulstes Signal (P) zu erzeugen und das Stimulationssignal (S) als gepulstes Signal (P) zu erzeugen.

9. Medizinisches Gerät (1) nach einem der vorhergehenden Ansprüche, wobei der Signalgenerator (2) dazu eingerichtet ist, das Kontrollsignal (K) als gepulstes Signal (P) zu erzeugen und das Stimulationssignal (S) als kontinuierliches Signal (C) zu erzeugen.

10. Medizinisches Gerät (1) nach Anspruch 9, wobei das Kontrollsignal (K) eine Impulsbreite (tP) von 0,01 ms bis 20 ms, bevorzugt von 0,05 ms bis 10 ms, weiter bevorzugt von 0,1 ms bis 1 ms, aufweist.

11. Medizinisches Gerät (1) nach Anspruch 9 oder 10, wobei der Signalgenerator (2) dazu eingerichtet ist, das Kontrollsignal (K) in wenigstens einem Burst (B1, B2, B3) mit einer Impulsanzahl von 1 bis 100 Impulsen, bevorzugt von 1 bis 10 Impulsen, weiter bevorzugt von 1 bis 5 Impulsen, zu erzeugen.

12. Medizinisches Gerät (1) nach Anspruch 11, wobei der Signalgenerator (2) dazu eingerichtet ist, das Kontrollsignal (K) in wenigstens zwei aufeinanderfolgenden Bursts (B1, B2; B2, B3) zu erzeugen, wobei eine Pausendauer (tR) zwischen den Bursts (B1, B2; B2, B3) wenigstens 10 s, bevorzugt wenigstens 15 s, weiter bevorzugt wenigstens 20 s, beträgt.

13. Medizinisches Gerät (1) nach einem der vorhergehenden Ansprüche, wobei der Signalgenerator (2) dazu eingerichtet ist, das Kontrollsignal (K) und das Stimulationssignal (S) alternierend zu erzeugen.

14. Medizinisches Gerät (1) nach einem der Ansprüche 1 bis 12, wobei der Signalgenerator (2) dazu eingerichtet ist, das Kontrollsignal (K) und das Stimulationssignal (S) gleichzeitig zu erzeugen.

15. Verfahren (10) zur Abstandskontrolle einer Elektrode (4) bei einer Schmerztherapie mittels elektrischer Neurostimulation, aufweisend die Schritte:
Erzeugen (11) eines elektrischen Kontrollsignals (K), wobei das Kontrollsignal (K) mittels eines Signalgenerators (2) eines medizinischen Geräts (1) erzeugt wird;
Abgeben (12) des erzeugten Kontrollsignals (K), wobei das Kontrollsignal (K) über eine mit dem Signalgenerator (2) verbundene Elektrode (4) einer Invasivkomponente (3) des medizinischen Geräts (1) abgegeben wird, wobei die Elektrode (4) in einem Abstand (G) zu einem Nerv (N) angeordnet ist, und wobei das Kontrollsignal (K) unter Einwirkung auf den Nerv (N) eine motorische und/oder sensorische Reaktion (R) auslöst, deren Ausprägung von dem Abstand (G) abhängig ist;
Erfassen (13) der Ausprägung der motorischen und/oder sensorischen Reaktion (R), wobei die motorische und/oder sensorische Reaktion (R) mittels einer Erfassungseinrichtung des medizinischen Geräts und/oder durch medizinisches Personal erfasst wird;
Kontrollieren (14) des Abstands (G) der Elektrode (4) in Abhängigkeit der Ausprägung der erfassten motorischen und/oder sensorischen Reaktion (R).
